# EUROPÄISCHE PATENTSCHRIFT

(11) **EP 3 463 089 B1**
(45) Veröffentlichungstag und Bekanntmachung des Hinweises auf die Patenterteilung: **07.10.2020**
(21) Anmeldenummer: 17724491.0
(22) Anmeldetag: 12.05.2017
(51) Int. Cl.: A61B 5/22, A61B 5/053

(54) **VORRICHTUNGSANORDNUNG ZUR ERFASSUNG KÖRPERLICHER LEISTUNGSWERTE EINES PROBANDEN SOWIE VERFAHREN ZUR ERMITTLUNG KÖRPERLICHER LEISTUNGSWERTE EINES PROBANDEN**
DEVICE ASSEMBLY FOR DETECTING PHYSICAL PERFORMANCE VALUES OF A TEST SUBJECT, AND METHOD FOR DETERMINING PHYSICAL PERFORMANCE VALUES OF A TEST SUBJECT
ENSEMBLE DE DISPOSITIFS POUR DÉTECTER DES VALEURS DE PERFORMANCES PHYSIQUES D'UN SUJET DE TEST ET PROCÉDÉ DE DÉTERMINATION DE VALEURS DE PERFORMANCES PHYSIQUE D'UN SUJET DE TEST

(30) Priorität: 24.05.2016 DE 102016006329
(43) Veröffentlichungstag der Anmeldung: 10.04.2019
(73) Patentinhaber: Fox, Robin, 29479 Jameln (DE)
(72) Erfinder: Fox, Robin, 29479 Jameln (DE)
(74) Vertreter: Hofmeister, Frank
(86) Internationale Anmeldenummer: PCT/EP2017/000580
(87) Internationale Veröffentlichungsnummer: WO 2017/202487

(56) Entgegenhaltungen:
- EP-A1- 1 413 250
- EP-A1- 1 902 675
- DE-U1-202016 002 157
- US-A- 5 579 782
- US-A1- 2004 059 242
- US-A1- 2011 065 550
- US-A1- 2012 245 436
- US-A1- 2013 317 386

## Beschreibung

Die Erfindung betrifft eine Vorrichtungsanordnung zur Erfassung körperlicher Leistungswerte eines Probanden mit einer Kraftmesseinrichtung sowie mit mindestens einer mit der Kraftmesseinrichtung verbindbaren Auswerteeinheit, wobei die Kraftmesseinrichtung mindestens einen Kraftsensor aufweist und als mobile tragbare Kraftmesseinrichtung so ausgebildet ist, dass der Proband von zwei einander entgegengesetzten Richtungen Kraft auf den mindestens einen Kraftsensor ausüben kann und, wobei die vom Probanden auf den mindestens einen Kraftsensor ausgeübte Kraft auf einer Anzeige der mindestens einen Auswerteeinheit darstellbar ist. Weiterhin betrifft die Erfindung ein Verfahren zur Ermittlung körperlicher Leistungswerte eines Probanden sowie eine Verwendung für die ermittelten Leistungswerte.

Um beim Training der körperlichen Fitness und insbesondere der körperlichen Muskelkraft einerseits das Training an den jeweiligen körperlichen Leistungsstatus des Probanden anpassen zu können und andererseits die Trainingsergebnisse überprüfen zu können, ist es unumgänglich zu Beginn des Trainings sowie fortlaufend während des Trainings die körperliche Muskelkraft des Probanden möglichst genau zu ermitteln.

In der Praxis erfolgt die Ermittlung dieses Leistungsstatus des Probanden ganz einfach dadurch, dass der Proband beispielsweise mit einem Arm gegen den Arm des Trainers drückt, der das Widerlager für die Kraftausübung des Probanden bildet. Diese sehr einfache Methode erlaubt aber nur eine sehr ungenaue Ermittlung des Leistungsstatus, da diese subjektiv von der Gegenhaltekraft des Trainers abhängig ist und darüber hinaus kaum reproduzierbar ist.

Eine tragbare Vorrichtung zur Ermittlung der Muskelkraft eines Probanden ist aus der US 4 307 608. Mit dieser bekannte Vorrichtung ist es zwar möglich, die absolute Muskelkraft eines Probanden pro Kraftübung zu ermitteln, eine Aussage über die tatsächliche Muskelkraft pro Muskelmasse, wie sie für ein effektives Krafttraining wünschenswert ist, ist mit dieser Vorrichtung aber nicht ermittelbar.

Eine weitere objektive Methode zur Ermittlung der körperlichen Muskelkraft eines Probanden ermöglicht der "Back-chek by Dr. Wolff ®". Bei dieser Methode wird der Proband in einem rahmenartigen Gestell aufgestellt und mittels verschiedener verschiebbarer Stempel positioniert. In verschiedenen Positionen kann der Proband dann über verschiedene Körperteile Druck auf die am Körper anliegenden Stempel ausüben. Die einzelnen Stempel sind mit Drucksensoren ausgestattet, so dass die vom Probanden auf die jeweiligen Stempel ausgeübten Kräfte objektiv ermittelt und gespeichert werden können.

Diese bekannte Methode ermöglicht zwar eine objektive Ermittlung der körperlichen Muskelkraft eines Probanden, jedoch ist der apparative Aufwand für dieses bekannte Verfahren sehr groß und somit auch mit einem hohen Kostenaufwand verbunden. Auch diese bekannte Vorrichtung dient ausschließlich dazu, die absolute Muskelkraft eines Probanden pro Kraftübung zu ermitteln. Weitere Geräte zur Erfassung der Körperzusammensetzung und/oder der Körperkraft sind aus folgenden Schriften bekannt: EP 1 902 675 A1, US 2012/0245436 A1, DE 20 2016 002 157 U1, US 5,579,782, US 2013/0317386 A1 und US 2004/0059242 A1.

Davon ausgehend liegt der Erfindung die **Aufgabe** zugrunde, eine Vorrichtungsanordnung zur Erfassung körperlicher Leistungswerte eines Probanden zu schaffen, die bei einfachem Aufbau und einfacher Handhabung die Ermittlung und Darstellung individueller reproduzierbarer Leistungswerte eines Probanden ermöglicht.

Die **Lösung** dieser Aufgabenstellung ist erfindungsgemäß dadurch gekennzeichnet, dass die Kraftmesseinrichtung zusätzlich Sensoren aufweist, über die zumindest der Körperfettgehalt des Probanden ermittelbar ist, und dass die Auswerteeinheit mindestens einen Microcontroller (MCU) zur gemeinsamen Auswertung der von der Kraftmesseinrichtung sowie den zusätzlichen Sensoren ermittelten Werte des Probanden beinhaltet.

Durch die Kombination der zur Ermittlung der Muskelkraft dienenden Kraftmesseinrichtung mit Sensoren, die zumindest der Ermittlung des Körperfettgehalts des Probanden dienen, ist es erstmalig mittels der zugehörigen Auswerteeinheit möglich, nicht nur die absolute Muskelkraft des Probanden zu ermitteln, sondern diese auch in ein Verhältnis zur Muskelmasse des Probanden zu setzen.

Durch die Ausstattung der Auswerteeinheit mit einem Microcontroler (MCU) ist es erstmalig möglich, die von dem Kraftsensor der Kraftmesseinrichtung sowie den zusätzlichen Sensoren gelieferten Werte in Echtzeit auszuwerten, darzustellen und dem Probanden eine Rückkopplung über seinen Leistungsstand zu liefern. Vorteilhafterweise können über die mindestens eine Auswerteeinheit ohne zusätzliche Geräte mehrere Kraftmesseinrichtungen miteinander gekoppelt werden, um auch komplexe und eventuell vergleichende Analysen eines oder mehreren Probanden durchführen zu können.

Mit einer praktischen Ausführungsform der Erfindung wird vorgeschlagen, dass an der Kraftmesseinrichtung beidseitig jeweils ein Griff angeordnet ist und die zusätzlichen Sensoren, über die zumindest der Körperfettgehalt des Probanden ermittelbar ist, an beiden Griffen angeordnet sind. Diese Ausgestaltung ermöglicht eine kompakte Zusammenfassung einer Vorrichtung zur Ermittlung der Muskelkraft mit einer Vorrichtung zur Erfassung weiterer individueller Körperwerte.

Zusätzlich wird mit der Erfindung vorgeschlagen, dass die die Kraftmesseinrichtung der Vorrichtungsanordnung eine vom Probanden zu betretenden Bodenplatte umfasst, an der die Kraftmesseinrichtung, beispielsweise zum Ausüben von die Zugkraft betreffenden Kraftübungen, einseitig festlegbar ist und, wobei in der Bodenplatte ebenfalls die zusätzlichen Sensoren angeordnet sind, über die zumindest der Körperfettgehalt des Probanden ermittelbar ist.

Durch diese Kombination der tragbaren Kraftmesseinrichtung mit der Bodenplatte lässt sich einerseits das Spektrum der vom Probanden ausübbaren Kraftübungen vergrößern und andererseits ermöglicht die Kombination der zusätzlichen Sensoren an den Griffen der Kraftmesseinrichtung und an der Bodenplatte, über die zumindest der Körperfettgehalt des Probanden ermittelbar ist, eine nach Körperregionen differenzierte Ermittlung der über die zusätzlichen Sensoren ermittelbaren Werte.

Bei der Verwendung der Kraftmesseinrichtung nur über die beidseitigen Griffe ermitteln die zusätzlichen Sensoren an den Griffen die Werte im wesentlichen nur für die Körperregion Arm-Schulter-Brustkorb, wohingegen bei der zusätzlichen Verwendung der Bodenplatte eine Messung quasi durch den gesamten Körper des Probanden erfolgt.

Gemäß einer bevorzugten Ausführungsform der Erfindung wird vorgeschlagen, dass der der mindestens eine Kraftsensor als Zug-Druck-Sensor oder Torsions-Sensor ausgebildet ist. Die Ausgestaltung des Kraftsensors als Zug-Druck-Sensor hat den Vorteil, dass der Proband unterschiedliche Muskelgruppen mit nur einer Vorrichtung ansprechen und testen kann. Bei der Verwendung der Zug-Komponente des Zug-Druck-Sensors übt der Proband auf die beiden einander entgegensetzten Seiten des Kraftsensors Zugkräfte aus, in dem die Vorrichtung quasi wie ein Expander benutzt wird.

Bei der Verwendung der Druck-Komponente des Zug-Druck-Sensors übt der Proband auf eine Seite des Kraftsensors eine Druckkraft auf, während sich die entgegengesetzte Seite des Kraftsensors als Widerlager am Probanden, beispielsweise am Brustkorb, abstützt.

Auf die Kraftmesseinrichtung ausgeübte Drehkräfte lassen sich durch den als Torsions-Sensor ausgestalteten Kraftsensor messen.

Mit einer praktischen Ausführungsform der Erfindung wird vorgeschlagen, dass der Kraftsensor der Kraftmesseinrichtung sowie die zusätzlichen Sensoren mittels USB-Verbindung oder Bluetooth mit der Auswerteeinheit verbunden sind, so dass auf eine störende Verkabelung der Kraftmesseinrichtung mit der Auswerteeinheit verzichtet werden kann.

Weiterhin wird mit der Erfindung vorgeschlagen, dass die zusätzlichen Sensoren zur Durchführung der Bioelektrischen Impedanzanalyse (BIA) ausgelegt sind. Die seit den 1980-er Jahren verbreitete Bioelektrische Impedanzanalyse dient zur Bestimmung der Körperzusammensetzung eines Probanden. Messbare Kennwerte der BIA sind die Körperanteile an Körperwasser, fettfreier Masse, Magermasse, Fettmasse, Körperzellmasse sowie extrazelluläre Masse. Die Messung erfolgt derart, dass mit einem konstanten Wechselstromsignal der Widerstand (die Impedanz) des Körpers des Probanden gemessen wird, wobei die zusätzlichen Sensoren dazu dienen, das Wechselstromsignal in den Körper einzuleiten und auch das Antwortsignal zu empfangen und an die Auswerteeinheit weiterzuleiten.

Weiterhin betrifft die Erfindung ein Verfahren zur Ermittlung körperlicher Leistungswerte eines Probanden. Das Verfahren ist gekennzeichnet durch die Verfahrensschritte:
a) Durchführen von mindestens drei verschiedenen Kraftübungen unter Verwendung der Kraftmesseinrichtung und Erfassen jeweils eines Kraftwertes pro Kraftübung mittels des mindestens einen Kraftsensors der Kraftmesseinrichtung;
b) Ermitteln eines Durchschnittskraftwertes aus den Einzelwerten des Verfahrensschrittes a) in der Auswerteeinheit;
c) Ermitteln zumindest des Körperfettgehalts des Probanden mittels der zusätzlichen Sensoren;
d) Ermitteln der Muskelmasse des Probanden aus den Werten des Verfahrensschrittes c) und dem in die Auswerteeinheit eingegebenen Körpergewicht des Probanden mittels der Auswerteeinheit und
e) Ermitteln des Leistungswertes Kraft pro Muskelmasse aus den Werten der Verfahrensschritte b), c) und d) mittels der Auswerteeinheit.

Im Verfahrensschritt a) führt der Proband mit der Kraftmesseinrichtung verschiedene Kraftübungen aus, wie beispielsweise Zugübungen vor der Brust und hinter dem Rücken sowie Druckübungen mit und ohne Benutzung der Bodenplatte. Zur Ermittlung eines Durchschnittskraftwertes werden die Kraftwerte der einzelnen Kraftübungen addiert und durch die Anzahl der Kraftübungen geteilt.

Durch die Kombination des Durchschnittskraftwertes mit den Sensoren, die zumindest der Ermittlung des Körperfettgehalts des Probanden dienen, ist es erstmalig mittels der zugehörigen Auswerteeinheit möglich, nicht nur die absolute Muskelkraft des Probanden zu ermitteln, sondern diese auch in ein Verhältnis zur Muskelmasse des Probanden zu setzen.

Das Ermitteln zumindest des Körperfettgehalts des Probanden mittels der zusätzlichen Sensoren erfolgt im Verfahrensschritt c) erfindungsgemäß über die zusätzlichen Sensoren beider Griffe der Kraftmesseinrichtung sowie der zusätzlichen Sensoren der Bodenplatte.

Aus der Ermittlung zumindest des Körperfettgehalts des Probanden mittels der zusätzlichen Sensoren in den beiden Griffen der Kraftmesseinrichtung und der Ermittlung zumindest des Körperfettgehalts des Probanden mittels der zusätzlichen Sensoren in einem Griff der Kraftmesseinrichtung und den zusätzlichen Sensoren der Bodenplatte wird mittels der Auswerteeinheit ein Mittelwert ermittelt.

Die Kombination der zusätzlichen Sensoren an den Griffen der Kraftmesseinrichtung und an der Bodenplatte, über die zumindest der Körperfettgehalt des Probanden ermittelbar ist, ermöglichen eine nach Körperregionen differenzierte Ermittlung der über die zusätzlichen Sensoren ermittelbaren Werte.

Schließlich wird mit der Erfindung vorgeschlagen, dass mittels der zusätzlichen Sensoren die für die Bioelektrische Impedanzanalyse (BIA) relevanten Körperwerte ermittelt werden.

Weiterhin betrifft die Erfindung die Verwendung der nach dem erfindungsgemäßen Verfahren ermittelten körperlichen Leistungswerte zum individuellen Training einzelner Muskelgruppen eines Probanden mittels der erfindungsgemäßen Vorrichtungsanordnung. Um die ermittelten Leistungswerte für ein reproduzierbares und für den Probanden leicht nachvollziehbares individuelles Muskeltraining verwenden zu können, wird mit der Erfindung vorgeschlagen, die ermittelten maximalen Leistungswerte für jede Muskelgruppe separat auf der Anzeige darstellbar sind und mittels der Kraftmesseinrichtung zu Trainingszwecken individuell vorgebbare Zwischenwerte ausübbar und auf er Anzeige visualisierbar sind.

Weitere Merkmale und Vorteile der Erfindung ergeben sich anhand der zugehörigen Zeichnungen, in denen zwei Ausführungsbeispiele einer erfindungsgemäßen Vorrichtungsanordnung zur Erfassung körperlicher Leistungswerte eines Probanden nur beispielhaft dargestellt sind, ohne die Erfindung auf diese Ausführungsbeispiele zu beschränken. In den Zeichnungen zeigt:
- Fig. 1: einen schematischen Darstellung einer erfindungsgemäßen Vorrichtungsanordnung zur Erfassung körperlicher Leistungswerte eines Probanden eines Probanden mit einem Längsschnitt durch eine erste Ausführungsform einer Kraftmesseinrichtung;
- Fig. 2: eine Darstellung gemäß Fig. 1, jedoch mit einer um 90° um die Längsachse gedrehten Kraftmesseinrichtung;
- Fig. 3: eine schematischen Darstellung einer erfindungsgemäßen Vorrichtungsanordnung mit einer Bodenplatte und
- Fig. 4: eine Darstellung gemäß Fig. 1, jedoch eine zweite Ausführungsform einer Kraftmesseinrichtung darstellend.

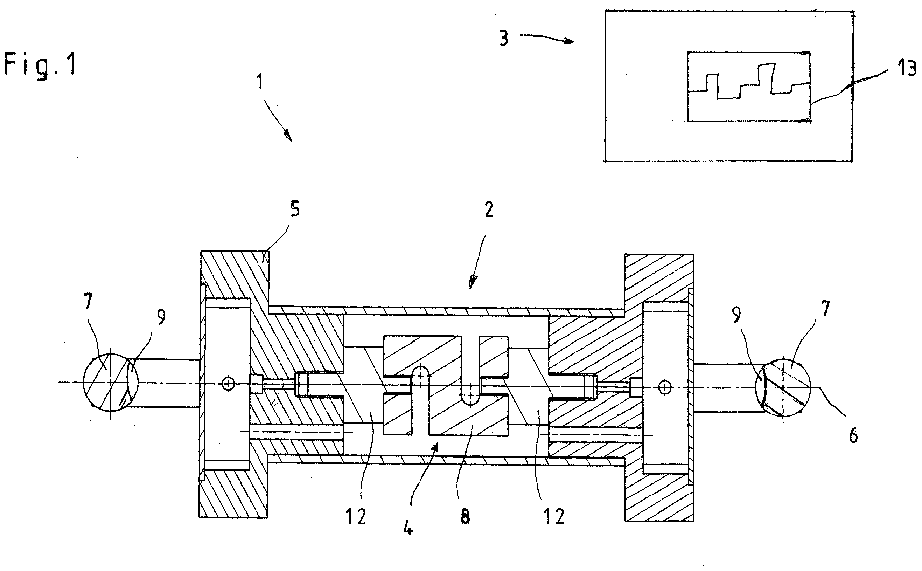

Die Abbildungen Fig. 1 bis 4 zeigen eine Vorrichtungsanordnung 1 zur Erfassung körperliche Leistungswerte eines Probanden. Eine derartige Vorrichtungsanordnung 1 wird verwendet, um beim Training der körperlichen Fitness und insbesondere der körperlichen Muskelkraft einerseits das Training an den jeweiligen körperlichen Leistungsstatus des Probanden anpassen zu können und andererseits die Trainingsergebnisse überprüfen zu können.

Die dargestellte Vorrichtungsanordnung 1 besteht im Wesentlichen aus einer Kraftmesseinrichtung 2 zur Erfassung der körperliche Leistungswerte eines Probanden sowie einer mit der Kraftmesseinrichtung 2 verbundenen Auswerteeinheit 3 zur Auswertung und Darstellung der vom Probanden an der Kraftmesseinrichtung 2 ermittelten Leistungs- und Körperwerte.

Die Kraftmesseinrichtung 2 besteht aus einem Kraftsensor 4, der in einem Gehäuse 5 angeordnet ist. Der Kraftsensor 4 ist dabei als mobiler tragbarer Kraftsensor 4 ausgebildet auf den der Proband von zwei einander entgegengesetzten Richtungen Kraft ausüben kann.

Um von zwei einander entgegengesetzten Richtungen Kraft auf den Kraftsensor 4 ausüben zu können, weist die Kraftmesseinrichtung 2 entlang der Längsachse 6 betrachtet an beiden Enden des Gehäuses 5 jeweils einen Griff 7 auf, wobei die Griffe 7 vorteilhafterweise gelenkig am Gehäuse 5 gelagert sind, um einen oder beide Griffe 7 auch mal ganz einklappen zu können und, um dem Probanden in jeder Lage der Handhabung der Kraftmesseinrichtung 2 eine bestmögliche Kraftausübung auf den Kraftsensor 4 zu ermöglichen.

Bei der Darstellung gemäß den Abbildungen Fig. 1 bis 4 ist der Kraftsensor 4 der Kraftmesseinrichtung 2 als Zug-Druck-Sensor 8 ausgebildet, der auf der Längsachse 6 zwischen den Griffen 7 im Gehäuse 5 angeordnet ist.

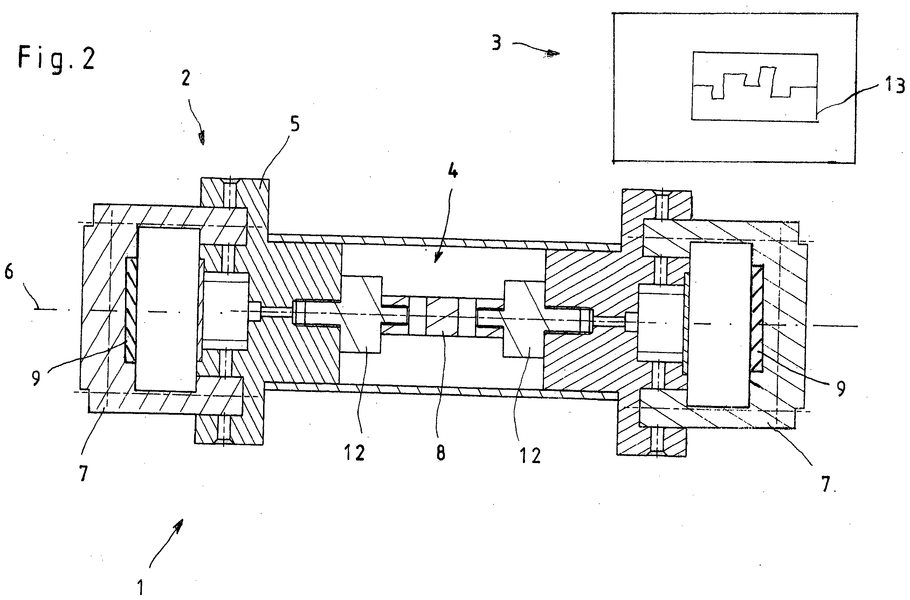

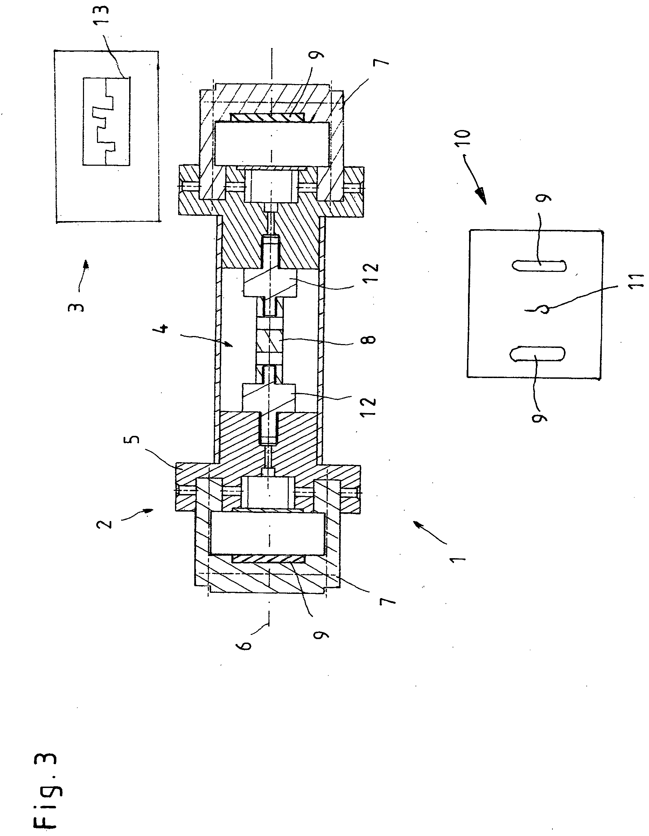

Wie insbesondere aus Fig. 2 und 3 ersichtlich, sind an den Griffen 7 der Kraftmesseinrichtung 2 Sensoren 9 angeordnet, über die zumindest der Körperfettgehalt des Probanden ermittelbar ist.

Vorteilhafterweise sind die Sensoren 9 so ausgelegt, dass sie zur Durchführung der Bioelektrischen Impedanzanalyse (BIA) geeignet sind, die die Bestimmung der Körperzusammensetzung eines Probanden ermöglicht.

Messbare Kennwerte der BIA sind die Körperanteile an Körperwasser, fettfreier Masse, Magermasse, Fettmasse, Körperzellmasse sowie extrazelluläre Masse. Die Messung erfolgt derart, dass mit einem konstanten Wechselstromsignal der Widerstand (die Impedanz) des Körpers des Probanden gemessen wird, wobei die Sensoren 9 dazu dienen, das Wechselstromsignal in den Körper einzuleiten und auch das Antwortsignal zu empfangen und an die Auswerteeinheit 3 weiterzuleiten.

Um einerseits das Spektrum der vom Probanden mittels der Kraftmesseinrichtung 2 ausübbaren Kraftübungen zu vergrößern und andererseits zu ermöglichen, dass über die Sensoren 9, über die zumindest der Körperfettgehalt des Probanden ermittelbar ist, eine nach Körperregionen differenzierte Ermittlung der Werte des Probanden erfolgen kann, weist die Vorrichtungsanordnung 1 zusätzlich eine vom Probanden zu betretende Bodenplatte 10 auf, wie dies der Abbildung Fig. 3 zu entnehmen ist. In Fig. 3 ist die Bodenplatte 3 nur schematisch und nicht maßstabsgerecht mit Bezug auf die Kraftmesseinrichtung 2 dargestellt.

Die Bodenplatte 10 eine Haltevorrichtung 11 auf, in die die Kraftmesseinrichtung 2 einseitig, beispielsweise über einen Griff 7, einhängbar ist, so dass der Proband auf einfache Art und Weise Zugübungen ausüben kann. Die Bodenplatte 10 mit dem sich bei der Übung darauf befindlichen Probanden bildet bei diesen Zugübungen das Widerlager für den am freien Griff 7 der Kraftmesseinrichtung 2 ziehenden Probanden.

Wie weiterhin aus Fig. 3 ersichtlich, ist auch die Bodenplatte 10 mit den Sensoren 9 ausgestattet, über die zumindest der Körperfettgehalt des Probanden ermittelbar ist. Im Falle der Bodenplatte 10 sind zwei im Wesentlichen parallel zueinander angeordnete Sensoren 9 vorgesehen, die vom Probanden barfüßig zu betreten sind.

Bei der Verwendung der Kraftmesseinrichtung 2 nur über die beidseitigen Griffe 7 ermitteln die Sensoren 9 an den Griffen 7 die Werte im Wesentlichen nur für die Körperregion Arm-Schulter-Brustkorb, wohingegen bei der zusätzlichen Verwendung der Bodenplatte 10 eine Messung quasi durch den gesamten Körper des Probanden von den Füßen bis zu den Händen erfolgt.

Die Abbildungen Fig. 1, 2 und 4 zeigen zwei verschiedene Ausführungsformen zum konstruktiven Aufbau der Kraftmesseinrichtung 2.

Bei der in den Abbildungen Fig. 1 und 2 dargestellten ersten Ausführungsform der Kraftmesseinrichtung 2 erfolgt der Anschluss der Griffe 7 an den Kraftsensor 4 über spielfrei zwischen das Gehäuse 5 und den Kraftsensor 4 zwischengeschaltete Kopplungsstücke 12.

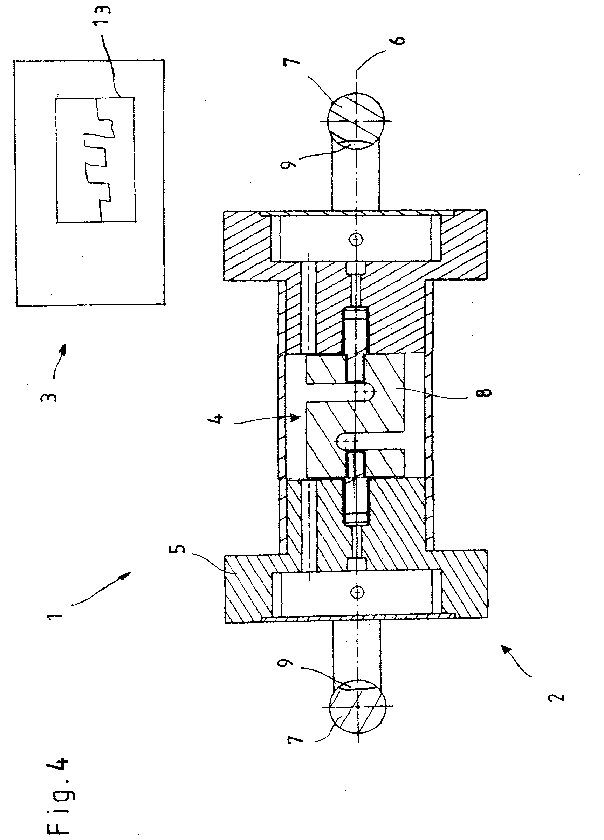

Eine kompaktere Bauweise der Kraftmesseinrichtung 2 zeigt die in Abbildung Fig. 4 dargestellte zweite Ausführungsform zur Ausbildung der Kraftmesseinrichtung 2, bei der das mit den Griffen 7 verbundene Gehäuse 5 direkt mit dem Kraftsensor 4 verbunden ist.

Alternativ zur Ausbildung des Kraftsensors 4 als einzelner Zug-Druck-Sensor 8 ist es selbstverständlich auch möglich, mehr als einen Kraftsensor 4 in dem Gehäuse 5 anzuordnen, oder aber den Kraftsensor 4 als reinen Druck-Sensor, Zug-Sensor oder Torsions-Sensor auszubilden.

Die verwendeten Kraftsensoren 4 sind vorzugsweise als Federkörper-Kraftsensoren 4 ausgebildet, deren Federkörper aufgrund der Krafteinwirkung durch den Probanden elastisch verformt wird. Die Verformung des Federkörpers wird durch Dehnungsmessstreifen, deren elektrischer Widerstand sich mit der Dehnung ändert, in die Änderung einer elektrischen Spannung umgewandelt. Über einen Messverstärker wird die elektrische Spannung und damit die Dehnungsänderung registriert.

Zur optischen und graphischen Darstellung dieser ermittelten Dehnungsänderung, die proportional zur vom Probanden auf den Kraftsensor 4 ausgeübten Kraft ist, sowie zur Übermittlung der mittels der Sensoren 9 an den Griffen 7 und der Bodenplatte 10 ist die Kraftmesseinrichtung 2, vorzugsweise mittels Bluetooth, mit der Auswerteeinheit 3 verbunden. Die optische Darstellung der gemessenen und ermittelten Werte erfolgt über mindestens eine Anzeige 13 der Auswerteeinheit 3.

Zur Verarbeitung der vom Kraftsensor 4 der Kraftmesseinrichtung 2 sowie den Sensoren 9 gelieferten Signale weist die Auswerteeinheit 3 mindestens einen Microcontroller (MCU) auf. Mit einer geeigneten Software lassen sich die vom Kraftsensor 4 sowie den Sensoren 9 gelieferten Signale entsprechend aufarbeiten und individuelle Leistungswerte des Probanden erstellen. Alternativ oder zusätzlich kann die Übertragung zwischen der Kraftmesseinrichtung 2 und der Auswerteeinheit 3 auch mittels eines USB-Anschlusses erfolgen.

Die Kopplung von Kraftmesseinrichtung 2 und Auswerteeinheit 3 mittels Bluetooth ist für den Probanden besonders vorteilhaft, da auf eine externe Verkabelung der Vorrichtung 1 verzichtet werden kann, die die Handhabung behindern könnte.

Die Energieversorgung für die Bluetooth-Übertragung sowie für die Verarbeitung der von dem Kraftsensor 4 erzeugten Signale erfolgt über eine Batterie oder einen Akku, der im Gehäuse 5 der Kraftmesseinrichtung 2, vorzugsweise im Bereich der Griffe 7, angeordnet ist. Das Einschalten der Energieversorgung kann beispielsweise über einen am Gehäuse 5 angeordneten Magnetschalter erfolgen.

Das Betätigen der voran beschriebenen Vorrichtungsanordnung 1 zur Erfassung körperliche Leistungswerte eines Probanden sowie die Ermittlung individueller Leistungswerte des Probanden geschieht wie folgt:
Nach dem Einschalten der Energieversorgung ergreift der Proband die Kraftmesseinrichtung 2, um verschiedenen Kraftübungen auszuführen, bei denen der Kraftsensor 4 der Kraftmesseinrichtung 2 auf Zug oder Druck belastet wird.

Beispielsweise ergreift der Proband die Kraftmesseinrichtung mit beiden Händen an den endseitig am Gehäuse 5 angeordneten Griffen 7 und zieht die beiden Griffe 7 in einander entgegengesetzte Richtungen auseinander. Diese Übung kann vor dem Brustkorb und auch hinter dem Rücken ausgeführt werden, um verschiedene Muskelgruppen anzusprechen und demzufolge verschiedene Kraftwerte zu ermitteln. Für eine weitere Kraftübung, bei der der Kraftsensor 4 auf Zug belastet wird, wird die Kraftmesseinrichtung 2 einseitig über einen Griff 7 in di Haltevorrichtung 11 der Bodenplatte 10 eingehängt und mit einer oder zwei Händen am anderen freien Griff 7 der Kraftmesseinrichtung 2 gezogen.

Die gelenkige Lagerung der Griffe 7 am Gehäuse 5 ermöglicht es dem Probanden immer eine optimale Griff- und/oder Haltestellung zur Kraftmesseinrichtung 2 einzunehmen, um die aufzubringende Kraft möglichst verlustfrei auf den Kraftsensor 4 übertragen zu können.

Für Druckbelastungen des Kraftsensors 4 ergreift der Proband die Kraftmesseinrichtung mit beiden Händen an den endseitig am Gehäuse 5 angeordneten Griffen 7 und drückt die beiden Griffe 7 in einander entgegengesetzte Richtungen aufeinander zu. Diese Kraftübung kann beispielsweise auch so durchgeführt werden, dass die Kraftmesseinrichtung 2 mit eingeklappten Griffen 7 zwischen den Oberschenkel des Probanden angeordnet wird und dieser durch Zusammendrücken der Oberschenkel Druck auf die beidseitigen Enden der Kraftmesseinrichtung 2 ausübt.

Die vom Probanden auf die am Gehäuse 5 gelagerten Griffe 7 ausgeübten Zug- und Druckkräfte werden direkt (Fig. 4), oder über die spielfrei zwischen den Griffen 7 und dem Zug-Druck-Sensor 8 angeordneten Kopplungsstücke 11 (Fig. 1 und 2) auf den Zug-Druck-Sensor 8 übertragen, wodurch dessen Federkörper aufgrund der Krafteinwirkung elastisch verformt wird. Die Verformung des Federkörpers wird durch Dehnungsmessstreifen, deren elektrischer Widerstand sich mit der Dehnung bzw. Stauchung ändert, in die Änderung einer elektrischen Spannung umgewandelt. Über einen Messverstärker wird die elektrische Spannung und damit die Dehnungsänderung registriert.

Die Spannungsänderungen werden mittels der Bluetooth- oder USB-Übertragung an die Auswerteeinheit 3 übermittelt und beispielsweise auf der Anzeige 13 graphisch dargestellt und/oder können zu weiteren Verwendung abgespeichert und mittels geeigneter Software ausgewertet und bewertet werden.

Zusätzlich oder alternativ zur Übertragung der ermittelten Werte an die Auswerteeinheit 3 ist es auch möglich, die Signale drahtgebunden oder drahtlos an einen externen PC oder ein Smartphone zu übermitteln.

Bei jeder der verschiedenen Kraftübung wird ein Kraftwert an die Auswerteeinheit 3 übermittelt.

In einem ersten Verfahrensschritt zur Ermittlung eines individuellen Leistungswertes des Probanden werden die Kraftwerte der einzelnen Kraftübungen addiert und durch die Anzahl der Kraftübungen geteilt, um einen Durchschnittskraftwert zu ermitteln.

Über die in den Griffen 7 sowie der Bodenplatte 10 angeordneten Sensoren 9 werden gleichzeitig mit der Ermittlung der Kraftwerte über der Kraftsensor 4 die Körperwerte des Probanden, wie zumindest der Körperfettgehalt, ermittelt und an die Auswerteeinheit 3 übermittelt. Vorteilhafterweise werden über die Sensoren 9 alle messbaren Kennwerte der Bioelektrischen Impedanzanalyse (BIA) erfasst, nämlich die Körperanteile an Körperwasser, fettfreier Masse, Magermasse, Fettmasse, Körperzellmasse sowie extrazellulärer Masse.

Aus der Ermittlung zumindest des Körperfettgehalts des Probanden mittels der Sensoren 9 in den beiden Griffen 7 sowie der Sensoren 9 in einem Griff 7 der Kraftmesseinrichtung 2 und den Sensoren 9 der Bodenplatte 10 wird mittels der Auswerteeinheit 2 ein Mittelwert ermittelt.

Die Kombination der zusätzlichen Sensoren an den Griffen der Kraftmesseinrichtung 2 und an der Bodenplatte 10, über die zumindest der Körperfettgehalt des Probanden ermittelbar ist, ermöglichen eine nach Körperregionen differenzierte Ermittlung der über die Sensoren 9 ermittelbaren Werte.

In einem weiteren Verfahrensschritt zur Ermittlung des individuellen Leistungswertes des Probanden lässt sich aus den über die Sensoren 9 ermittelten Körperwerte des Probanden unter Bezugnahme auf das in die Auswerteeinheit 3 eingebbare Körpergewicht des Probanden die Muskelmasse in Prozent des Körpergewichts oder in Kilogramm Masse bestimmen.

Durch Verknüpfung dieser ermittelten Muskelmasse des Probanden mit dem zuvor ermittelten Durchschnittskraftwert ergibt dies einen individuellen Kraftwert pro Kilogramm Muskelmasse des Probanden.

Da die Kenntnis nur der reinen Muskelmasse keine Aussage über die tatsächliche Muskelkraft des Probanden erlaubt, ist es mit der voranstehend beschriebenen Vorrichtungsanordnung 1 sowie dem Verfahren zur Ermittlung des individuellen Leistungswertes erstmalig möglich, einen reproduzierbaren individuellen Kraftwert pro Kilogramm Muskelmasse zu ermitteln.

Dieser ermittelte Kraftwert ermöglicht die Erstellung und Überprüfung individueller Trainingsprogramme sowohl zum Muskelaufbau nach Krankheit oder Unfall als auch zum reinen Krafttraining.

Die nach dem voranstehenden Verfahren ermittelten körperlichen Leistungswerte können, wie nachstehend beschrieben, zum individuellen Training einzelner Muskelgruppen eines Probanden mittels der Kraftmesseinrichtung 2 verwendet werden.

Um die ermittelten Leistungswerte für ein reproduzierbares und für den Probanden leicht nachvollziehbares individuelles Muskeltraining verwenden zu können, werden zunächst die ermittelten maximalen Leistungswerte für jede Muskelgruppe separat, beispielsweise als Balkendiagramme, auf der Anzeige 13 dargestellt.

Da es weder beim Krafttraining noch bei dem Wiederaufbau der Muskulatur nach Krankheit oder Unfall sinnvoll ist, die Muskulatur immer zu 100% zu beanspruchen, ermöglicht die Visualisierung der Leistungsgrenzen einen gezielten und dosierbaren Einsatz der Kraftmesseinrichtung 2. Anhand der in Echtzeit mittels der Auswerteeinheit 3 ermittelten und auf der Anzeige 13 dargestellten Leistungswerte für die in der jeweiligen Übung beanspruchte Muskelgruppe kann der Proband genau erkennen wieviel Prozent seiner Leistungsfähigkeit er zum jetzigen Zeitpunkt abruft bzw. einsetzt.

Diese Trainingsmethode ermöglicht ein propriozeptives Training der Tiefenmuskulatur, das für alle Menschen mit motorischen Störungen ein dosiertes individuelles Training verschiedener Muskelgruppen bietet.

Auf diese Weise lassen sich individuelle Trainingsprogramme erstellen, die vom Probanden aufgrund der einfachen bildlichen Darstellung gut zu überprüfen ist.

Mittels einer geeigneten Software lassen sich in der Auswerteeinheit 3 die von der Kraftmesseinrichtung 2 und den Sensoren 9 gelieferten Signale in Echtzeit direkt auswerten, so dass Minimalwerte, Maximalwerte, Durchschnittswerte sowie Kraft- und Energiebilanzen über die Zeit ermittelbar, darstellbar und abspeicherbar sind. Diese Messungen ermöglichen eine reproduzierbare und vergleichbare Messung, die unabhängig von Personeneinflüssen, wie beispielsweise Ablesezeitpunkt und Protokollierungsfehlern, ist.

Auch sind mehrere Kraftmesseinrichtungen 2 mit einer Auswerteeinheit 3 zu einem Verbund zusammenschaltbar, um auch eine komplexe simultane Analytik zu ermöglichen.

Weiterhin ermöglicht die Kopplung der Kraftmesseinrichtung 2 mit der Auswerteeinheit 3 auch den Ablauf von vorprogrammierten Trainingsprogrammen, wobei beispielsweise über die Auswerteeinheit 3 ein "Kraftwert" vorgegeben wird, der mit der Kraftmesseinrichtung 2 zu erzielen ist.

Eine solchermaßen ausgestaltete Vorrichtung 1 ermöglicht bei einfachem Aufbau und einfacher Handhabung die Ermittlung individueller reproduzierbarer Leistungswerte eines Probanden.

### Bezugszeichenliste

- 1: Vorrichtungsanordnung
- 2: Kraftmesseinrichtung
- 3: Auswerteeinheit
- 4: Kraftsensor
- 5: Gehäuse
- 6: Längsachse
- 7: Griff
- 8: Zug-Druck-Sensor
- 9: Sensor
- 10: Bodenplatte
- 11: Haltevorrichtung
- 12: Kopplungsstücke
- 13: Anzeige

## Patentansprüche

1. Vorrichtungsanordnung zur Erfassung körperliche Leistungswerte eines Probanden mit einer Muskelkraftmesseinrichtung (2) sowie mit mindestens einer mit der Muskelkraftmesseinrichtung (2) verbindbaren Auswerteeinheit (3), wobei die Muskelkraftmesseinrichtung (2) mindestens einen Kraftsensor (4) und beidseitig jeweils einen Griff (7) aufweist, so dass der Proband über die beiden Griffe (7) von zwei einander entgegengesetzten Richtungen Muskelkraft auf den mindestens einen Kraftsensor (4) ausüben kann und wobei die Muskelkraftmesseinrichtung (2) als mobile tragbare Muskelkraftmesseinrichtung (2) so ausgebildet ist, dass die vom Probanden auf den mindestens einen Kraftsensor (4) ausgeübte Muskelkraft auf einer Anzeige (13) der mindestens einen Auswerteeinheit (3) darstellbar ist und die Muskelkraftmesseinrichtung (2) Sensoren (9) aufweist, über die zumindest der Körperfettgehalt des Probanden ermittelbar ist, und dass die Auswerteeinheit (3) mindestens einen Microcontroller (MCU) zur gemeinsamen Auswertung der von der Muskelkraftmesseinrichtung (2) sowie den Sensoren (9) ermittelten Werte des Probanden beinhaltet, wobei die Sensoren (9) an beiden Griffen (7) angeordnet sind.

2. Vorrichtungsanordnung nach Anspruch 1, **dadurch gekennzeichnet, dass** die Muskelkraftmesseinrichtung (2) eine vom Probanden zu betretenden Bodenplatte (10) umfasst, an der die Muskelkraftmesseinrichtung (2) einseitig festlegbar ist und, wobei in der Bodenplatte (10) ebenfalls die Sensoren (9) angeordnet sind, über die zumindest der Körperfettgehalt des Probanden ermittelbar ist.

3. Vorrichtungsanordnung nach Anspruch 1 oder 2, **dadurch gekennzeichnet, dass** der mindestens eine Kraftsensor (4) der Muskelkraftmesseinrichtung (2) als Zug-Druck-Sensor (8) oder Torsions-Sensor ausgebildet ist.

4. Vorrichtungsanordnung nach einem der Ansprüche 1 oder 2, **dadurch gekennzeichnet, dass** der Kraftsensor (4) der Muskelkraftmesseinrichtung (2) sowie die zusätzlichen Sensoren (9) mittels USB-Verbindung oder Bluetooth mit der Auswerteeinheit (3) verbunden sind.

5. Vorrichtungsanordnung nach einem der Ansprüche 1 bis 4, **dadurch gekennzeichnet, dass** die Sensoren (9) zur Durchführung der Bioelektrischen Impedanzanalyse (BIA) ausgelegt sind.

6. Verfahren zur Ermittlung körperlicher Leistungswerte anhand einer Vorrichtungsanordnung nach einem der Ansprüche 1 bis 5,
wobei folgende Verfahrensschritte ausgeführt werden:
a) Durchführen von mindestens drei verschiedenen Kraftübungen unter Verwendung der Muskelkraftmesseinrichtung (2) und Erfassen jeweils eines Kraftwertes pro Kraftübung mittels des mindestens einen Kraftsensors (4) der Muskelkraftmesseinrichtung (2);
b) Ermitteln eines Durchschnittskraftwertes aus den Einzelwerten des Verfahrensschrittes a) in der Auswerteeinheit (3);
c) Ermitteln zumindest des Körperfettgehalts des Probanden mittels der Sensoren (9);
d) Ermitteln der Muskelmasse des Probanden aus den Werten des Verfahrensschrittes c) und dem in die Auswerteeinheit (3) eingegebenen Körpergewicht des Probanden mittels der Auswerteeinheit (3) und
e) Ermitteln des Leistungswertes Kraft pro Muskelmasse aus den Werten der Verfahrensschritte b), c) und d) mittels der Auswerteeinheit (3).

7. Verfahren nach Anspruch 6, **dadurch gekennzeichnet, dass** das Ermitteln zumindest des Körperfettgehalts des Probanden mittels der zusätzlichen Sensoren (9) im Verfahrensschritt c) über die Sensoren (9) beider Griffe (7) der Muskelkraftmesseinrichtung (2) und der Sensoren (9) der Bodenplatte (10) erfolgt.

8. Verfahren nach Anspruch 7, **dadurch gekennzeichnet, dass** aus der Ermittlung zumindest des Körperfettgehalts des Probanden mittels der Sensoren (9) in den beiden Griffen (7) der Muskelkraftmesseinrichtung (2) und der Ermittlung zumindest des Körperfettgehalts des Probanden mittels der Sensoren (9) in einem Griff (7) der Muskelkraftmesseinrichtung (2) und den Sensoren (9) der Bodenplatte (10) mittels der Auswerteeinheit (3) ein Mittelwert ermittelt wird.

9. Verfahren nach einem der Ansprüche 6 bis 8, **dadurch gekennzeichnet, dass** mittels der Sensoren (9) die für die Bioelektrische Impedanzanalyse (BIA) relevanten Körperwerte ermittelt werden.

10. Verwendung der nach dem Verfahren gemäß der Ansprüche 6 bis 9 ermittelten körperlichen Leistungswerte zum individuellen Training einzelner Muskelgruppen eines Probanden mittels der Vorrichtungsanordnung nach einem der Ansprüche 1 bis 6,
**dadurch gekennzeichnet,**
**dass** die ermittelten maximalen Leistungswerte für jede Muskelgruppe separat auf der Anzeige (13) darstellbar sind und mittels der Muskelkraftmesseinrichtung (2) zu Trainingszwecken individuell vorgebbare Zwischenwerte ausübbar und auf der Anzeige (13) visualisierbar sind.

## Claims

1. Device assembly for detecting physical performance values of a test subject, with a muscle strength meter (2) and with at least one evaluation unit (3) connectable to the muscle strength meter (2), wherein the muscle strength meter (2) has at least one strength sensor (4) and on each side has a grip (7), such that the test subject can apply muscle strength to the at least one strength sensor (4) from two opposite directions via the two grips (7), and wherein the muscle strength meter (2) is designed as a mobile portable muscle strength meter (2), such that the muscle strength applied to the at least one strength sensor (4) by the test subject can be indicated on a display (13) of the at least one evaluation unit (3), and the muscle strength meter (2) has sensors (9) via which at least the body fat content of the test subject can be determined, and the evaluation unit (3) contains at least one microcontroller (MCU) for joint evaluation of the test subject values determined by the muscle strength meter (2) and by the sensors (9), wherein the sensors (9) are arranged at both grips (7).

2. Device assembly according to Claim 1, **characterized in that** the muscle strength meter (2) comprises a floor plate (10) onto which the test subject steps, and on which the muscle strength meter (2) can be fixed at one end, wherein the sensors (9) via which at least the body fat content of the test subject can be determined are likewise arranged in the floor plate (10).

3. Device assembly according to Claim 1 or 2, **characterized in that** the at least one strength sensor (4) of the muscle strength meter (2) is designed as a tension/compression sensor (8) or torsion sensor.

4. Device assembly according to either of Claims 1 and 2, **characterized in that** the strength sensor (4) of the muscle strength meter (2) and the additional sensors (9) are connected to the evaluation unit (3) by means of a USB connection or Bluetooth.

5. Device assembly according to one of Claims 1 to 4, **characterized in that** the sensors (9) are designed to carry out bioelectrical impedance analysis (BIA).

6. Method for determining physical performance values using a device assembly according to one of Claims 1 to 5, in which method the following method steps are carried out:
a) at least three different strength exercises are carried out using the muscle strength meter (2) and, for each strength exercise, a strength value is detected by means of the at least one strength sensor (4) of the muscle strength meter (2);
b) an average strength value is determined in the evaluation unit (3) from the individual values from method step a);
c) at least the body fat content of the test subject is determined by means of the sensors (9) ;
d) the evaluation unit (3) determines the muscle mass of the test subject from the values from method step c) and from the test subject body weight input into the evaluation unit (3) and
e) the strength per muscle mass is determined from the values from method steps b), c) and d) by means of the evaluation unit (3).

7. Method according to Claim 6, **characterized in that** the determination of at least the body fat content of the test subject by means of the additional sensors (9) in method step c) is effected via the sensors (9) of both grips (7) of the muscle strength meter (2) and the sensors (9) of the floor plate (10).

8. Method according to Claim 7, **characterized in that** an average value is determined by means of the evaluation unit (3) from the determination of at least the body fat content of the test subject by means of the sensors (9) in the two grips (7) of the muscle strength meter (2) and from the determination of at least the body fat content of the test subject by means of the sensors (9) in a grip (7) of the muscle strength meter (2) and the sensors (9) of the floor plate (10).

9. Method according to one of Claims 6 to 8, **characterized in that** the body values relevant for the bioelectrical impedance analysis (BIA) are determined by means of the sensors (9).

10. Use of the performance values determined by the method according to Claims 6 to 9 for individual training of individual muscle groups of a test subject, by means of the device assembly according to one of Claims 1 to 6, **characterized in that** the determined maximum performance values can be shown on the display (13) separately for each muscle group, and interim values that can be predefined individually for training purposes can be applied by means of the muscle strength meter (2) and viewed on the display (13).

## Revendications

1. Arrangement de système destiné à acquérir des valeurs de puissance physiques d'un sujet, comprenant un dispositif de mesure de la force musculaire (2) ainsi qu'au moins une unité d'interprétation (3) qui peut être reliée au dispositif de mesure de la force musculaire (2), le dispositif de mesure de la force musculaire (2) possédant au moins un capteur de force (4) et respectivement une poignée (7) de chaque côté, de sorte que le sujet peut exercer une force musculaire sur l'au moins un capteur de force (4) par le biais des deux poignées (7) depuis deux directions mutuellement opposées et le dispositif de mesure de la force musculaire (2) étant réalisé sous la forme d'un dispositif de mesure de la force musculaire (2) portable mobile de sorte que la force musculaire exercée par le sujet sur l'au moins un capteur de force (4) peut être représentée sur un afficheur (13) de l'au moins une unité d'interprétation (3) et le dispositif de mesure de la force musculaire (2) possédant des capteurs (9) par le biais desquels il est possible de déterminer au moins la teneur en graisse corporelle du sujet, et l'unité d'interprétation (3) contenant au moins un microcontrôleur (MCU) destiné à l'interprétation commune des valeurs du sujet déterminées par le dispositif de mesure de la force musculaire (2) ainsi que par les capteurs (9), les capteurs (9) étant disposés sur les deux poignées (7).

2. Arrangement de système selon la revendication 1, **caractérisé en ce que** le dispositif de mesure de la force musculaire (2) comporte un socle (10) sur lequel doit monter le sujet, d'un côté duquel peut être fixé le dispositif de mesure de la force musculaire (2), les capteurs (9) par le biais desquels peut être déterminée au moins la teneur en graisse corporelle du sujet étant également disposés dans le socle (10).

3. Arrangement de système selon la revendication 1 ou 2, **caractérisé en ce que** l'au moins un capteur de force (4) du dispositif de mesure de la force musculaire (2) est réalisé sous la forme d'un capteur de traction-pression (8) ou d'un capteur de torsion.

4. Arrangement de système selon l'une des revendications 1 ou 2, **caractérisé en ce que** l'au moins un capteur de force (4) du dispositif de mesure de la force musculaire (2) ainsi que les capteurs (9) supplémentaires sont reliés à l'unité d'interprétation (3) par le biais d'une liaison USB ou du Bluetooth.

5. Arrangement de système selon l'une des revendications 1 à 4, **caractérisé en ce que** les capteurs (9) sont conçus pour réaliser l'analyse d'impédance bioélectrique (BIA).

6. Procédé de détermination de valeurs de puissance physiques à l'aide d'un arrangement de système selon l'une des revendications 1 à 5, les étapes suivantes étant exécutées :
a) réalisation d'au moins trois exercices de force différents en utilisant le dispositif de mesure de la force musculaire (2) et acquisition respectivement d'une valeur de force par exercice de force au moyen de l'au moins un capteur de force (4) du dispositif de mesure de la force musculaire (2) ;
b) détermination d'une valeur de force moyenne à partir des valeurs individuelles de l'étape a) dans l'unité d'interprétation (3) ;
c) détermination d'au moins une teneur en masse corporelle du sujet au moyen des capteurs (9) ;
d) détermination, au moyen de l'unité d'interprétation (3), de la masse musculaire du sujet à partir des valeurs de l'étape c) et du poids corporel du sujet saisi dans l'unité d'interprétation (3) et
e) détermination de la valeur de puissance force par masse musculaire à partir des valeurs des étapes b), c) et d) au moyen de l'unité d'interprétation (3).

7. Procédé selon la revendication 6, **caractérisé en ce que** la détermination d'au moins la teneur en masse corporelle du sujet au moyen des capteurs (9) à l'étape c) est effectuée par le biais des capteurs (9) des deux poignées (7) du dispositif de mesure de la force musculaire (2) et des capteurs (9) du socle (10).

8. Procédé selon la revendication 7, **caractérisé en ce qu'**une valeur moyenne est déterminée au moyen de l'unité d'interprétation (3) à partir de la détermination d'au moins la teneur en masse corporelle du sujet au moyen des capteurs (9) dans les deux poignées (7) du dispositif de mesure de la force musculaire (2) et de la détermination d'au moins la teneur en masse corporelle du sujet au moyen des capteurs (9) dans une poignée (7) du dispositif de mesure de la force musculaire (2) et les capteurs (9) dans le socle (10).

9. Procédé selon l'une des revendications 6 à 8, **caractérisé en ce que** les valeurs corporelles pertinentes pour l'analyse d'impédance bioélectrique (BIA) sont déterminées au moyen des capteurs (9).

10. Utilisation des valeurs de puissance physique déterminées conformément au procédé selon l'une des revendications 6 à 9 pour l'entraînement personnalisé de groupes de muscles individuels d'un sujet au moyen d'un arrangement de système selon l'une des revendications 1 à 6,
**caractérisée en ce que**
les valeurs de puissance maximales déterminées peuvent être représentées sur l'afficheur (13) séparément pour chaque groupe de muscles et des valeurs intermédiaires peuvent être prédéfinies de manière personnalisée au moyen du dispositif de mesure de la force musculaire (2) à des fins d'entraînement et peuvent être visualisées sur l'afficheur (13).
